(19) Europäisches Patentamt

European Patent Office

Office européen des brevets

(11) **EP 1 382 966 A1**

(12) **EUROPEAN PATENT APPLICATION**

(43) Date of publication:
21.01.2004 Bulletin 2004/04

(51) Int Cl.⁷: **G01N 33/543**

(21) Application number: 02026815.7

(22) Date of filing: 28.11.2002

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: 03.07.2002 US 188527

(71) Applicant: **CHEN, Hsin-Huang**
**Kaohsiung (TW)**

(72) Inventor: **CHEN, Hsin-Huang**
**Kaohsiung (TW)**

(74) Representative: **Viering, Hans-Martin, Dipl.-Ing.**
**Patent- und Rechtsanwälte**
**Viering, Jentschura & Partner,**
**Steindorfstrasse 6**
**80538 München (DE)**

(54) **Microvolume detecting method and device**

(57) The invention provides a microvolume assay method of an affinity reaction using a membrane of a small area in a microvolume reaction container for performing said affinity reaction between a target substance in a sample and a receptor capable of binding to the target substance, which method comprises the steps of:

(1) taking a microvolume of the sample and loading to the membrane to allow the target substance in the sample to attach to the membrane;

(2) placing the membrane into the microvolume reaction container, adding a solution containing an antibody (receptor) capable of binding to the target substance, wherein the volume of the solution is sufficient to soak the entire area of the membrane attaching the sample;

(3) incubating the membrane in the reaction container for a time sufficient to allow the target substance attached to the membrane to bind to the receptor by an affinity reaction to form a target substance-receptor complex; and

(4) detecting the target substance-receptor complex.

A device for performing the microvolume assay method of the invention is also provided.

EP 1 382 966 A1

**Description**

**FIELD OF THE INVENTION**

This invention relates to a method and device for carrying out microvolume assays. In particular, the invention provides an assay for affinity reaction, such as an immunoassay.

**BACKGROUND OF THE INVENTION**

Affinity reaction analysis technique indicators, carried out by reacting receptors and target substance(s), have been developed for years to determine the existence of target substances. Radioactive labels, fluorescent or other chromogenic indicators are used as labels to determine the presence of target substances, e.g. immunoassay of antigen specifically binding to antibody.

Recently, the use of enzyme, colored latex particles, and metallic colloids have been considered a simple and rapid technique for detection, and the results can be evaluated with naked eyes by non-technical people. However, convenient and rapid methods have several disadvantages such as low sensitivity and low accuracy. In particular, it is difficult to obtain a desirable result when determining extremely low concentrations of the target substance in a sample.

A dot-immunobinding assay for monoclonal and other antibodies has been disclosed. The principle of such type of assay is as follows: A diluted solution or suspension of antigen is dotted on a nitrocellulose piece of filter paper, and the dot is incubated with test antibody and with peroxide-conjugated second antibody directed against the first antibody. A positive reaction is detected as a colored dot. The advantages of this assay are that (1) the amounts of the antigen and antibody needed for the assay are low, and (2) the nitrocellulose paper provides an almost white background color, so that the positive reaction color is easily observed. See Analytical Biochemistry, 119, 142-147 (1982). However, the disadvantages of the immunoassay are (1) complicated techniques involving more than 20 steps of dilution, incubation, and washing to carry out the assay, and (2) it is very time-consuming. A standard assay takes over 20 hours to perform.

USP 4,774,177 disclosed another immunoassay for detecting the presence of antibody or antigen in a sample. This method utilizes a nitrocellulose carrier having a primary ligand (haptin or antibody ) stably bound thereto a microsized spot, about three mm in diameter. The nitrocellulose carrier is treated with a liquid containing non-specific globulin for the primary ligand in order to block binding sites other than the antibody binding sites of the primary ligand. The liquid sample (about 50μl) suspected of containing the antibody is applied to the spot on the nitrocellulose carrier. Then the nitrocellulose carrier is incubated at room temperature for a time sufficient to bind any antibody that may be present in the sample, to the primary ligand to form a ligand-antibody complex. This takes about six minutes. A liquid containing labeled antiglobulin is then added to the complex and incubated at room temperature for about three minutes to bind the labeled antiglobulin to the antibody-ligand complex. The nitrocellulose carrier is washed and ready to be applied to a suitable color developer to complete the assay. However, the steps of the method are complicated. If no container is provided, and the liquid is directly added to the nitrocellulose carrier to form the antiglobulin-antibody-ligand complex, the liquid usually dries out during incubation.

This invention overcomes many of the shortcomings of more conventional methods and provides a more convenient, more rapid, and more sensitive method for handling microvolume detection.

**DESCRIPTION OF THE INVENTION**

The invention provides a microvolume assay method of an affinity reaction using a membrane of a small area in a microvolume reaction container for performing said affinity reaction between a target substance in a sample and a receptor capable of binding to the target substance, which method comprises the steps of:

(1) taking a microvolume of the sample and loading it to the membrane to allow the target substance in the sample to attach to the membrane;

(2) placing the membrane into the microvolume reaction container, adding a solution containing an antibody capable of binding to the target substance, wherein the volume of the solution is sufficient to soak the entire area of the membrane carrying the sample;

(3) incubating the membrane in the reaction container for a time sufficient to allow the target substance attached on the membrane to bind to the receptor by an affinity reaction to form a target substance-receptor complex; and

(4) detecting the target substance-receptor complex.

The other objective of the invention is to provide a device for performing the microvolume assay method of the invention.

## BRIEF DESCRIPTION OF THE DRAWING

Figure 1 is a graphical representation of fluid from healthy pumpkin tissue (A) and the test samples containing 6.4 (B), 3.2(C), 1.6(D), 0.8(E), 0.4(F), 0.2(G), 0.1 (H) ng/µl of zucchini yellow mosaic virusillustrated in Example 3. The amount of test sample applied to the method is 0.2 µl.

Figure 2 is a graphical representation of fluid from healthy pumpkin tissue (A) and the test samples containing 6.4 (B), 3.2(C), 1.6(D), 0.8(E), 0.4(F), 0.2(G) ng/µl of zucchini yellow mosaic virus illustrated in Example 4. The amount of test sample applied to the method is 0.5 µl.

## DETAILED DESCRIPTION OF THE INVENTION

The term "affinity reaction" is defined as a pair of molecules, where one of the molecules has an area on the surface or in a cavity which specifically binds to a particular spatial and polar organization of the other molecule. The members of the specific binding pair include, but are not limited to, antigen and antibody, complementary nucleic acid fragments, or a pair of proteins capable of recognizing a particular spatial and polar organization of each other.

The term "affinity reaction assay" is defined as an assay for carrying out the objective of affinity reaction analysis such as immunoassay or hybridization detection method. The embodiment of the invention is immunoassay which includes, but is not limited to, direct detection immunoassay or sandwich immunoassay. Desirably, the assay can employ a signal producing system for measuring the spectrophotometric property of affinity binding, e.g. enzyme immunoassay.

The term "target substance" refers to any substance capable of interacting with another substance which includes, but is not limited to, ligand, DNA, RNA antigen, pathogen, receptor protein, biotin or other substances combined with avidin or any other substance that has the specific affinity interaction with another substance.

The term "receptor" usually refers to any substance capable of recognizing and binding with the target substance. Illustrative receptors include, but are not limited to, DNAs, RNAs antibodies, antigens, haptins, nucleic acid fragments, proteins or protein fragments, receptor poteins, biotin or other substances combined with avidin or any other substance that has the specific affinity interaction with another substance.

The invention relates to a microvolume detection method. The characteristics of the method reside in that only a microvolume of test sample is needed for detection, and permit convenient, rapid and sensitive detection.

The theory of the invention is based on Michaelis-Menten Kinetics. Antigen-antibody complex is used as an example to explain the specific affinity interaction (test sample being as antigen).

Let: Ag = Antigen; Ab = Antibody; [O]= Concentration of the object O;

Complex = Complex formed by antigen-antibody specific recognition

(binding);

$$Ag+Ab \rightarrow complex$$

Then, the dissociation constant, Kd, can be represented as

$$K\text{d} = [Ag] \times [Ab]/[Complex]$$

For most antigen-antibody reactions, Kd is around $1 \times 10^{-9}$M, meaning that the two reactions, Ag and Ab, will tend to form complexes when the total concentration of reactants is equal to or greater than $1 \times 10^{-9}$M. Therefore, if

the concentration of antigen is very low, one can increase the concentration of Ab to still drive the reaction in favor of the formation of Ag-Ab complexes. With the special minimum-volume design, we can easily increase the effective concentration of the Ab without extra expense. In addition, with the increased concentration of the reactants, the speed and sensitivity of the test strips are also improved. This can be shown by the Michaelis-Menten equation as follows:

$$V=V_{max}[S]/([S]+K_m)$$

Where V and $V_{max}$ stand for the velocity and maximum velocity of the reaction, respectively; [S] stands for the concentration of the substrate, and $K_m$ stands for the dissociation constant of the reaction at the steady-states.
The theory of invention to improve the reaction rate can be illustrated by means of Michaelis-Menten equation:

$$V=V_{max}[Ag]/([Ag]+K_d)$$

Where V and $V_{max}$ stand for the velocity and maximum velocity of the affinity reaction, respectively; [Ag] stands for the concentration of the substrate, and $[K_d]$ stands for the dissociation constant of the affinity reaction.
By inverting the above equation, the Lineweaver-Burk equation can be obtained:

$$1/V=K_d/V_{max}[Ag]+1/V_{max}$$

Therefore, it can be derived from the equation that when the concentration of the substrate [Ag] is increased, $V_{max}[Ag]$ will approach the maximum; so $K_d/V_{max}[Ag]$ approaches zero, 1/V approaches $1/V_{max}$, that is, the velocity of the reaction V approaches $V_{max.}$. Hence, with our special design using a minimum volume of reactants and a higher concentration of labeled antibodies in the kits, both the speed and sensitivity of the detection approach the maximum.
The subject method differs from prior art methods by providing a method for determining extremely low concentrations of target substances through a simple and time-saving assay. Prior art immunoassay takes even 8 hours to perform the assay, while the method according to this invention allows for assays to be carried out in a few minutes, up to one hour. This invention is also superior to the prior art because of its simple protocol and convenient handling.
The invention provides a microvolume assay method of an affinity reaction using a membrane of a small area in a microvolume reaction container for performing said affinity reaction between a target substance in a sample and a receptor capable of binding to the target substance, which method comprising the steps of:

(1) taking a microvolume of the sample and loading it to the membrane to allow the target substance in the sample to attach to the membrane;

(2) placing the membrane into the microvolume reaction container, adding a solution containing an antibody capable of binding to the target substance, wherein the volume of the solution is sufficient to soak the entire area of the membrane carrying the sample;

(3) incubating the membrane in the reaction container for a time sufficient to allow the target substance attached to the membrane to bind to the receptor by an affinity reaction to form a target substance-receptor complex; and

(4) detecting the target substance-receptor complex.

The method and device according to the invention is suitable for any type of affinity interaction including, but not limited to, direct or sandwich immunoassay, nuclear acid hybridization, or other known affinity interaction analyses.
Preferably, this invention is useful for detection of any type of body fluids or tissues from animal, humans, and plants. According to the invention, fluid samples include, but are not limited to, human or animal body fluids or fluids in plant tissues. The test samples employed in this invention include, but are not limited to, antigen, antibody, pathogens, virus, nucleic acid fragments and protein fragments. It is desirable to remove the color of the background (e.g., green fluid from plant tissue or red fluid from blood sample). Discoloring reagent is any reagent that can remove the color of the background such as bleach, alcohol, chloroform, acetone, carbon tetrachloride, or a mixture thereof.
According to the invention, the membrane used to absorb the target substance of test sample may be any porous material capable of absorbing the target substance. Such materials include, but are not limited to, nitrocellulose, nylon, glass fiber, PVDF, rayon, filter paper, polyvinyl chloride, polyethylene, polystryene, diazotized paper, activated beads, protein A *(Staphylococcus aureus)* beads, polylysine, polyarginine, polyhistidine, glass and plastic. In a preferred embodiment of the invention, nitrocellulose is employed. The area of membrane is as small as possible so that the membrane can still bind the sample in an amount effective to form an observable spot when the tagged antiglobulin is assayed. The area is preferably between $1mm^2$ and $200mm^2$, e.g. $100mm^2$.

In accordance with the subject invention, a solid phase can advantageously be employed to substantially support the membrane, e.g. plastic strip. A wide variety of materials and shapes may be employed. In one embodiment of the invention, an integral part of strip or stick, such as a test strip, is employed to retain the said membrane on the end of the strip. The size of the solid phase should not be limited. For example, the width is less than 10mm, preferably less than 5mm. Said strip supporting the membrane can be made sharp to penetrate plant tissue, human body, or animal body surface so that the test sample can touch said membrane or the target substance of the test sample can be adsorbed onto the membrane.

In one embodiment of the invention, a strip whose width is or is less than 3mm is employed. In addition, a piece of membrane covers one end of the strip which is sharp to penetrate the plant tissue.

The amount of liquid test sample applied to the method of the invention is extremely small, preferably 0.5ul or less. A variety of methods of applying the test sample onto said membrane are known in the art and can be employed to practice the invention. For instance, one can drop or dot the test sample onto said membrane, and/or rinse the membrane into the test sample solution. In one embodiment of the invention, a sharp stick or needle is used to dot the liquid test sample onto the membrane. The observable spot is preferably between 0.2 and 1.5mm in diameter, more preferably less than 0.5mm in diameter.

The microvolume reaction container is used to conduct the affinity reaction according to this invention. There is no limitation on the shape of said container, as long as the volume is sufficient to rinse the membrane, the solid phase, or the strip with the reaction solution.

In one embodiment of the invention, the microvolume reaction container has a circle or half-circle opening or flask bottom or is divided into upper and lower portions. The diameter of lower portion is less than that of the upper portion.

According to the invention, the target substance and receptor undergoing affinity reaction is recommended in a solution to be in an amount sufficient to completely rinse the said membrane, e.g. 3-200μl, preferably with 20-50μl.

According to the invention, the membrane absorbed with test sample should be incubated for a period of time sufficient to ensure complete affinity reaction and target substance-receptor complex formation. The reaction volume is so small that it enables the high speed reaction. The reaction time can even be shortened to a few minutes, which is far less than that of the conventional methods.

In order to practice the invention, a receptor capable of binding the target substances can be obtained commercially or prepared by conventional techniques. For instance, antibodies can be prepared using conventional immunological techniques.

Detection of the target substance-receptor complex may be accomplished using known standard affinity reaction analysis. For instance, the detection can be carried out with labeled substances capable of producing signals. According to the invention, these labeled substances include, but are not limited to

    (1) enzyme, for example:

    -   horseradish peroxidase, examples of substrates are 1,1'-trimethylene-bis (4-formylpyridinium bromide) dioxime (TMB), 2,2'*o*-azinobis (3-ethylbenzthiazoline-sulfonic acid) (ABTS), *o*-phenylenediamine dihydrochloride (OPD), DAB/Cobalt, Chloronaphthol, or any fluorometric or cold light labels;

    -   Soybean Peroxidase, the substrates are mentioned as above;

    -   Alkaline Phosphatase, the substrates are *p*-Nitrophenyl phosphate (PNPP), nitro-blue tetrazolium/5 -bromo-4-chloro-3 '-indolylphosphate-*p*-toluidine salt (NBT/BCIP), Fast Red TR/AS and any fluorometric or cold light labels;

    -   β-Galactosidase, substrates are, for example, *o*-nitrophenyl-β-D-galactopyranoside (ONPG), isopropyl-β-D-thiogalactopyranoside/5-bromo-4-chloro-3'-indolyl-β-D-galactopyranoside (IPTG/X-Gal);

    -   Glucose Oxidase, substrates are NBT, phenazine methosulfate (PMS);

    (2) Biotine-Avidin;

    (3) colored latex particles;

(4) metallic colloid;

(5) flouroometric substances; or

(6) radioisotopes, for example, .sup. 125 -iodine and .sup. 35 - sulfur.

The subject invention achieves a simple technique for detecting a variety of target substance so that assays can be carried out without highly trained personnel. This purpose is of great importance when the detection measurement is by visual inspection. Particularly, the signals can be observed by naked eyes, preferably with color development, for example, peroxidase, colored latex particles and metallic colloid. Alkalinephosphatase is used for a preferred embodiment of the invention.

The conjugation of the label substance and the receptor may be performed prior to combining the label-bound-receptor with the target substance. The enzymes can be covalently bound to a specific anti-immunoglobulin, which in turn is bound to the antibody whose presence is to be determined.

In a preferred aspect, the invention relates to the "Sandwich Immunoassay," in which the target substances are bound with receptor and a second labeled receptor is added to produce signals.

In accordance with the subject invention, the target substance-receptor complexes should be carefully washed to remove any non-specifically bound target, receptor, and/or label substances. The washing buffer can be water, phosphate-buffered saline (PBS), Tris-buffered saline (TBS), or saline solution. A surfactant such as Tween-20, Triton-X 100 can be added if necessary.

According to the invention, after incubation for a sufficient time, signal generating compounds produce signals. To avoid the misreading of the result, stopping solution can be added to stop the reaction. For instance, after the enzyme and substrate interaction is completed, add stopping solution mentioned above or other stopping reagents to stop the reaction.

The dilution buffer, in accordance with the invention, can be the washing buffer with a small amount of protein, e.g. 0.5-15% of non-fat milk, 0.1-5% bovine serum albumin, egg albumine or the mixture thereof. A surfactant such as Tween-20, Triton-X 100 etc, can be added if necessary.

In a preferred embodiment of the invention, a microvolume assay method of an affinity reaction uses a membrane of a small area in a microvolume reaction container for performing said affinity reaction between a target substance in a sample and a receptor capable of binding to the target substance, which method comprises the steps of:

(1) taking a microvolume of the sample and loading it to the membrane to allow the target substance in the sample to attach to the membrane;

(2) optionally rinsing the membrane in discoloring solution for a sufficient time;

(3) placing said membrane into the microvolume reaction container, adding a solution containing a receptor capable of binding the target substance and labeled with a signal generating compound, wherein the volume of the solution is sufficient to soak the entire area of the membrane attaching the sample;

(4) incubating the membrane in the reaction container for a time sufficient to allow the target substance attached on the membrane to bind to the receptor through an affinity reaction to form a target substance-receptor complex;

(5) washing said membrane, which is treated in step (4), for a few times and removing the unbound receptor solution; and

(6) observing the signal and determining the existence of the target substance.

The detection assay can detect one or several target substances simultaneously by undergoing the affinity reaction with labeled receptor and target substance(s).

The subject invention also provides a method for detecting the presence of one or more target substances simultaneously in a single liquid test sample. One or several spots have been bound to the said membrane capable of binding to the first receptor. The dilution buffer is added to block the membrane and avoid interference. Said membrane is incubated with the second receptor. The method for detecting the presence of one or more than one target substances comprises the steps of:

(1) stably binding one or more spots of first receptor capable of binding with the target substances onto the membrane;

(2) blocking the membrane by adding the solution containing a small amount of protein;

(3) taking a microvolume of the sample and loading it to the spot(s) of the membrane;

(4) optionally rinsing the membrane adsorbed with the sample in discoloring solution for a time sufficient to discolor it;

(5) placing the membrane into the microvolume reaction container;

(6) adding 2-200µl second receptor solution capable of binding with target substance to the microvolume reaction container, and incubating the membrane in the reaction container for a time sufficient to allow the target substance attached on the membrane of the strip to bind to said receptor by an affinity reaction to form a target substance-receptor complex;

(7) if necessary, washing the membrane treated with step (6) for several times to remove the additional second receptor solution; and

(8) detecting the target substance-receptor complex.

The invention also provides a device for performing the microvolume assay method of an affinity reaction between a target substance in a sample and a receptor capable of binding to the target substance, which device comprises the components of:

(1) a membrane onto which the sample is to be loaded, so that the target substance can be adsorbed onto the membrane;

(2) a microvolume reaction container in which the target substance and the receptor can undergo the affinity reaction and form the target substance-receptor complex; and

(3) a component for use in detection of the target substance-receptor complex.

Further components may be employed to carry out the microvolume assay of affinity reaction, including

(4) micropipet;

(5) bottles containing other reagents; and

(6) control strip i.e., a membrane which has adsorbed a sufficient amount of target substance as compared to sample and to determine the presence of the target substance.

A further aspect of the invention provides a device for carrying out the affinity reaction assays of the invention, which comprises:

(1) test strip which is a strip or stick supporting a membrane capable of adsorbing the target substance;

(2) microvolume reaction container;

(3) a bottle containing reagent capable of binding to the receptor which is labeled or can be bound to a labeled substance;

(4) a bottle containing signal generating reagents; and, if necessary, further comprises

(5) micropipet;

(6) bottles containing other reagents; and

(7) control strip i.e., a membrane which has adsorbed a sufficient amount of target substance as compared to sample and to determine the presence of the target substance.

The following embodiment is offered by way of illustration and not by way of limitation.

### Example 1 Preparation of labeled antibody

Vigorously mix the purified zucchini yellow mosaic virus (1mg/mL) with complete or incomplete adjuvants injected into the New Zealand rabbit and rechallenge every seven days. The optimal antibody response to virus is obtained after one month. By means of ammonium sulphate precipitation and DEAE column chromatography separation, the rabbit anti-virus IgG is obtained and then conjugated with horseradish peroxidase. 1.5% bovine serum albumin and 0.1% Tween-20 phoso-buffered saline (PBS) solution are also added to the reaction solution.

### Example 2 Preparation of test strip

Cut the plastic strip into 3 mm. by 75mm., and attach a 3 mm. by 5mm. nitrocellulose to the plastic strip.

### Example 3 Microvolume reaction detection Assay

1 Take 0.2μl fluid from healthy pumpkin tissue and standard test samples containing 6.4, 3.2, 1.6, 0.8, 0.4, 0.2 and 0.1ng/μl zucchini yellow mosaic virus.

2 The above fluid from healthy pumpkin tissue and the standard test samples are then dotted on the membrane of test strip based on Example 2. Preferably, the diameter of the spot is less than 0.5mm.

3 Pipet 25μl of reaction solution prepared by Example 1 into a microvolume reaction container.

4 Rinse the strip which absorbs fluid from pumpkin tissue prepared from step 2 in the container and incubate it for 40 minutes.

5 Wash the strip with washing buffer.

6 Place the strip into the bottle containing color developer and incubate it for 7 minutes.

7 The green background of the strip can be removed by soaking the strip in discoloring solution. The strip is blotted with clean, absorbent paper to remove excess discoloring solution.

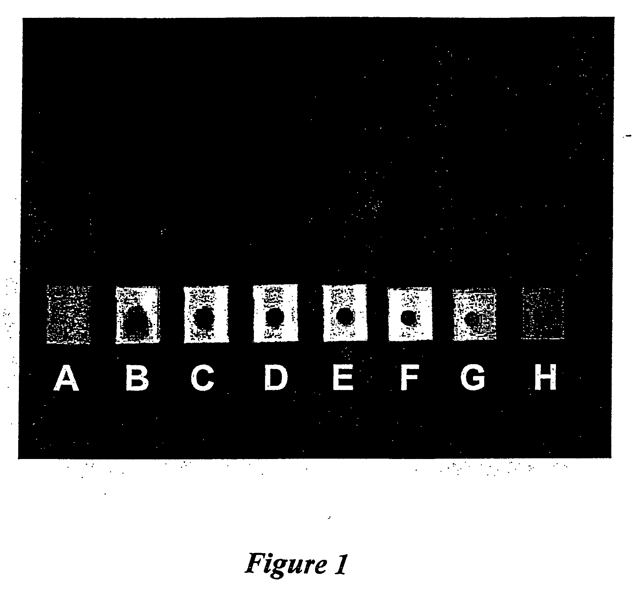

The result as shown in Figure 1 indicates that the fluid from healthy pumpkin tissue has no color change but the test samples containing 6.4ng/μl (B), 3.2ng/μl (C), 1.6ng/μl (D), 0.8ng/μl (E), 0.4ng/μl (F), 0.2ng/μl (G) and 0.1 ng/μl (H) of zucchini yellow mosaic virus have clear color development. The method permits a high sensitivity of detection which can reach 0.02ng.

### Example 4 Microvolume reaction detection Assay

1 Take 0.5μl fluid from healthy pumpkin tissue and standard test samples containing 6.4,-3.2, 1.6, 0.8, 0.4, 0.2ng/μl zucchini yellow mosaic virus.

2 The above fluid from healthy pumpkin tissue and the standard samples are then dotted on the membrane of test strip based on Example 2. Preferably, the diameter of the spot is less than 0.5mm.

3 The green background of the strip can be removed by soaking the strip in discoloring solution. The strip is blotted with clean, absorbent paper to remove excess discoloring solution.

4 Pipet 25μl of reaction solution prepared by Example 1 into a microvolume reaction container.

5 Rinse the strip which absorbs fluid from pumpkin tissue in the container and incubate it for 35 minutes to complete the reaction.

6 Wash the strip with washing buffer.

7 Place the strip into the bottle containing color developer and incubate it for 5 minutes.

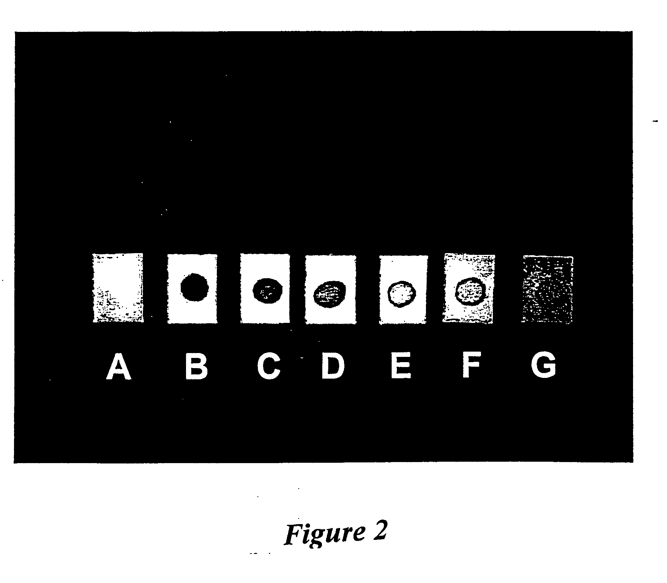

The result as shown in Figure 2 indicates that the fluid from healthy pumpkin tissue has no color change but the test samples containing 6.4ng/μl (B), 3.2ng/μl (C), 1.6ng/μl (D), 0.8ng/μl (E), 0.4ng/μl (F) and 0.2ng/μl (G) of pumpkin yellow mosaic virus have clear color development. The method permits a high sensitivity of detection which can reach 0.1ng.

**Claims**

1. A microvolume assay method of an affinity reaction using a membrane of a small area in a microvolume reaction container for performing said affinity reaction between a target substance in a sample and a receptor capable of binding to the target substance, which method comprises the steps of:

   (1) taking a microvolume of the sample and loading it to the membrane to allow the target substance in the sample to attach to the membrane;

   (2) placing the membrane into the microvolume reaction container, adding a solution containing an antibody capable of binding to the target substance, wherein the volume of the solution is sufficient to soak the entire area of the membrane attaching the sample;

   (3) incubating the membrane in the reaction container for a time sufficient to allow the target substance attached on the membrane to bind to the receptor by an affinity reaction to form a target substance-receptor complex; and

   (4) detecting the target substance-receptor complex.

2. The method according to Claim 1, further comprising the steps of:

   (1') prior to step (2), soaking the membrane in discoloring solution for a time sufficient to discolor it;

   (3') prior to step (4), soaking the membrane in discoloring solution for a time sufficient to discolor it; and

   (4') step (4) comprising the step which makes the label bound to the target substance-receptor complex produce the signal, and observe the signal to determine the existence of the target substance.

3. The method according to Claim 1, wherein 0.5ul of test sample is employed.

4. The method according to Claim 1, wherein the sample can be spotted on the membrane with a sharp stick or needle to form an observable spot.

5. The method according to Claim 4, wherein said spot is less than 1mm in diameter.

6. The method according to Claim 5, wherein said spot is less than 0.5mm in diameter.

7. The method according to Claim 1, wherein 3-200μl of receptor solution is employed.

8. The method according to Claim 7, wherein 20-50ul of receptor solution is employed.

9. The method according to Claim 1, wherein the membrane materials are selected from the group consisting of nitrocellulose, nylon, glass fiber, PVDF, rayon, filter paper, polyvinyl chloride, polyethylene, polystryene, diazotized paper, activated beads, protein A (Staphylococcus aureus) beads, polylysine, polyarginine, polyhistidine, glass and plastic.

10. The method according to Claim 1, wherein the membrane is nitrocellulose.

11. The method according to Claim 1, wherein the detection of said target substance-receptor complex is assayed by labeling the complex with a signal producing compound.

12. The method according to Claim 11, wherein the label is a color developer.

13. The method according to Claim 11, wherein the said label is an enzyme, colored latex particles, or metallic colloid.

14. The method according to Claim 12, wherein the said label is an enzyme.

15. The method according to Claim 1, wherein the sample is a liquid sample.

16. The method according to Claim 15, wherein the sample is plant tissue fluid.

17. The method according to Claim 15, wherein the sample is animal body fluid (animal serum, whole blood, plasma, or urine).

18. The method according to Claim 15, wherein the sample is microbial growth medium.

19. The method according to Claim 1, wherein'the microvolume reaction container has a circle, oral or half circle opening which is less than 10 mm in diameter.

20. The method according to Claim 19, wherein the microvolume reaction container has a flask bottom.

21. The method according to Claim 19, wherein the microvolume reaction container is divided into upper and lower portions, the lower portion having a diameter which is less than the diameter of the upper portion.

22. The method according to Claim 20, wherein the microvolume reaction container is divided into upper and lower portions, the lower portion having a diameter shorter than that of the upper protion.

23. The method according to Claim 1, wherein the membrane can be stick on a solid phase.

24. The method according to Claim 23. wherein said solid phase is a plastic strip.

25. The method according to Claim 1, which can be applied to detect one or more target substance(s) simultaneously.

26. The method according to Claim 25, which can utilize the receptor solution capable of binding to the target substance.

27. A method for detecting the presence of one or more of the target substance(s) by use of a membrane with a small area in a microvolume reaction container undergoing the reaction, which method comprises the steps of:

(1) stably binding one or more spots of first receptor capable of binding with the target substances onto the membrane;

(2) blocking the membrane;

(3) taking a microvolume of the sample and loading it to the spot(s) of the membrane;

(4) if necessary, rinsing the membrane adsorbed with the sample in discoloring solution for a time sufficient to discolor;

(5) placing the membrane treated with step (4) into the microvolume reaction container;

(6) adding 2-200ul second receptor solution capable of binding with the target substance to the microvolume reaction container and incubating the membrane in the reaction container for a time sufficient to allow the target substance attached to the membrane of the strip to bind to said receptor by an affinity reaction to form a target substance-receptor complex;

(7) if necessary, washing the membrane treated in step (6) several times to remove the additional second receptor solution; and

(8) detecting the target substance-receptor complex.

**28.** A device for performing the microvolume assay method of an affinity reaction between a target substance in a sample and a receptor capable of binding to the target substance as defined in Claim 1, which device comprises the components of:

(1) a membrane onto which the sample be loaded, so that the target substance can be adsorbed onto the membrane;

(2) a microvolume reaction container in which the target substance and the receptor can undergo the affinity reaction and form the target substance-receptor complex; and

(3) a component for use in detection of the target substance-receptor complex.

**29.** The device according to Claim 28, further comprising the following components:

(4) micropipet;

(5) bottles containing other reagents; and

(6) control strip i.e., a membrane which has adsorbed a sufficient amount of target substance as compared to sample and to determine the presence of the target substance.

**30.** The device according to Claim 28, comprising:

(7) test strip which is a rod or stick supporting a membrane capable of adsorbing the target substance;

(8) microvolume reaction container;

(9) a bottle containing reagents capable of binding to the receptor solution which is labeled or can be bound to a labeled substance;

(10) a bottle containing signal generating reagents; and if necessary, further comprising

(11) micropipet;

(12) bottles containing other reagents; and

(13) control strip i.e., a membrane which has adsorbed a sufficient amount of target substance as compared to sample and to determine the presence of the target substance.

**31.** The device according to Claim 28, wherein the membrane bound to the strip is selected from the group consisting of nitrocellulose, nylon, glass fiber, PVDF, rayon, filter paper, polyvinyl chloride, polyethylene, polystryene, diazotized paper, activated beads, protein A *(Staphylococcus* aureus) beads, polylysine, polyarginine, polyhistidine, glass and plastic.

**32.** The device according to Claim 28, wherein the membrane bound to the strip is nitrocellulose.

**33.** The device according to Claim 28, in which the detection of said target substance-receptor complex is assayed by labeling the complex with signal producing compound.

**34.** The device according to Claim 28, wherein the label is color developer.

**35.** The device according to Claim 34, wherein the label is an enzyme, colored latex particles, or a metallic colloid.

**36.** The device according to Claim 35, wherein the label is an enzyme.

*Figure 1*

*Figure 2*

**European Patent Office**

**EUROPEAN SEARCH REPORT**

Application Number

EP 02 02 6815

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 301 141 A (MICROBIOLOGICAL RESEARCH CORP) 1 February 1989 (1989-02-01)<br><br>* abstract *<br>* page 4, line 16 - line 45 *<br>* page 5, line 20 - line 29 *<br>* page 5, line 41 - line 46 *<br>* page 5, line 51 - line 58 *<br>* page 7, line 7 - line 13 *<br>--- | 1,7-15, 23, 25-28, 31-36 | G01N33/543 |
| A | US 4 774 177 A (MARKS JANET M) 27 September 1988 (1988-09-27)<br>* the whole document *<br>--- | 1-36 | |
| A | US 2002/132256 A1 (CAVANAUGH PHILIP GERARD) 19 September 2002 (2002-09-19)<br>* the whole document *<br>----- | 1-36 | |
| | | | **TECHNICAL FIELDS SEARCHED (Int.Cl.7)**<br><br>G01N |

The present search report has been drawn up for all claims

| Place of search | Date of completion of the search | Examiner |
|---|---|---|
| MUNICH | 24 November 2003 | Weijland, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                     EP 02 02 6815

This annex lists the patent family members relating to the patent documents cited in the above-mentioned European search report.
The members are as contained in the European Patent Office EDP file on
The European Patent Office is in no way liable for these particulars which are merely given for the purpose of information.

24-11-2003

| Patent document cited in search report | | Publication date | Patent family member(s) | | Publication date |
|---|---|---|---|---|---|
| EP 0301141 | A | 01-02-1989 | EP | 0301141 A1 | 01-02-1989 |
| US 4774177 | A | 27-09-1988 | NONE | | |
| US 2002132256 | A1 | 19-09-2002 | NONE | | |

For more details about this annex : see Official Journal of the European Patent Office, No. 12/82